# EUROPÄISCHE PATENTSCHRIFT

(11) **EP 2 544 744 B1**
(45) Veröffentlichungstag und Bekanntmachung des Hinweises auf die Patenterteilung: **07.01.2015**
(21) Anmeldenummer: 11719990.1
(22) Anmeldetag: 10.03.2011
(51) Int. Cl.: A61M 15/06, A61L 9/04

(54) **INHALATORKOMPONENTE**
INHALER COMPONENT
ÉLÉMENT D'INHALATEUR

(30) Priorität: 10.03.2010 AT 3852010
(43) Veröffentlichungstag der Anmeldung: 16.01.2013
(73) Patentinhaber: Batmark Limited, London WC2R 2PG (GB)
(72) Erfinder: BUCHBERGER, Helmut, A-4482 Ennsdorf (AT)
(74) Vertreter: Schrell, Andreas
(86) Internationale Anmeldenummer: PCT/AT2011/000122
(87) Internationale Veröffentlichungsnummer: WO 2011/109848

(56) Entgegenhaltungen:
- WO-A1-88/01884
- WO-A1-96/22801
- DE-A1-102006 039 115
- US-A- 3 521 643
- US-A- 5 501 236
- US-A- 5 636 787

## Beschreibung

Die Erfindung betrifft eine Inhalatorkomponente, aufweisend:
ein Gehäuse mit einem Gehäusemantel;
ein Mundstück mit einer Mundstücköffnung für die Zufuhr eines inhalierbaren Mediums in die Mundhöhle eines Benutzers;
ein mit der Umgebung durch Diffusion kommunizierbares Riechstoff-Reservoir, beinhaltend einen Riechstoff, zur Freisetzung des Riechstoffes in die Umgebung und zur olfaktorischen Wahrnehmung desselben durch den Benutzer.

In der gegenständlichen Patentanmeldung bezieht sich der Begriff "Inhalator" auf medizinische wie nicht-medizinische Inhalationsapparate. Der Begriff bezieht sich außerdem auf Zigarettenersatz-Artikel, wie sie beispielsweise in der Europäische Patentklasse A24F47/00B enthalten sind, soweit diese dazu bestimmt sind, dem Benutzer ein inhalierbares Medium darzureichen. Das inhalierbare Medium besteht aus einem Dampf-Luft-Gemisch oder/und Aerosol, insbesondere aus einem nikotinhaltigen Dampf-Luft-Gemisch oder/und Aerosol. Die Verwendung des Singulars "Riechstoff" soll nicht darauf schließen lassen, daß der Riechstoff nur aus einem Inhaltsstoff bestehe. Vielmehr kann der "Riechstoff" auch eine große Anzahl unterschiedlichster Einzelsubstanzen beinhalten. Als "ein mit der Umgebung durch Diffusion kommunizierbares Riechstoff-Reservoir" kommt an sich jedes Reservoir in Betracht, sofern sich der darin enthaltene Riechstoff durch Verdunstung oder/und Sublimation und Diffusion in die Umgebung verflüchtigen kann.

Heute verfügbare Nikotin-Inhalatoren sind in der Regel so konzipiert, daß sie ein nikotinhaltiges Dampf-Luft-Gemisch oder/ und Aerosol in die Mundhöhle eines Benutzers verabreichen. Als Beispiel sei der unter der Bezeichnung "Nicorette® Inhaler", vgl. www.nicorette.de, von der Firma McNeil Consumer Healthcare GmbH vertriebene Nikotin-Inhalator genannt. Dieser Inhalator führt dem Benutzer über ein Mundstück ein nikotinhaltiges Dampf-Luft-Gemisch zu, dessen organoleptischen Effekte von vielen Benutzern als unangenehm empfunden werden. In keinster Weise vermag dieser Inhalator die komplexen organoleptischen Effekte von Zigarettenrauch nachzubilden. Eine olfaktorische Reizstimulierung, wie sie beim Zigarettenrauchen während eines Zuges durch den vom glimmenden Ende der Zigarette abströmenden Nebenstromrauch bewirkt wird, unterbleibt komplett. Man kann davon ausgehen, daß dieser zusätzliche olfaktorische Stimulus mit einem Schlüsselreiz gleichzusetzen ist, welcher Zigarettenrauchern einen wesentlichen Anreiz vermittelt, den Tabakkonsum aufrechtzuerhalten.

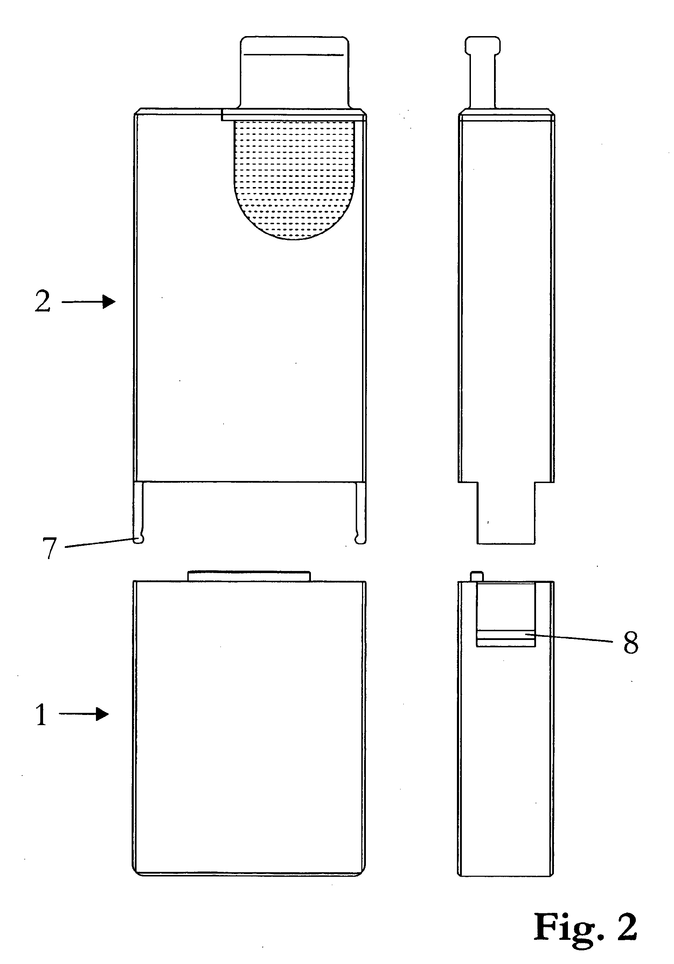

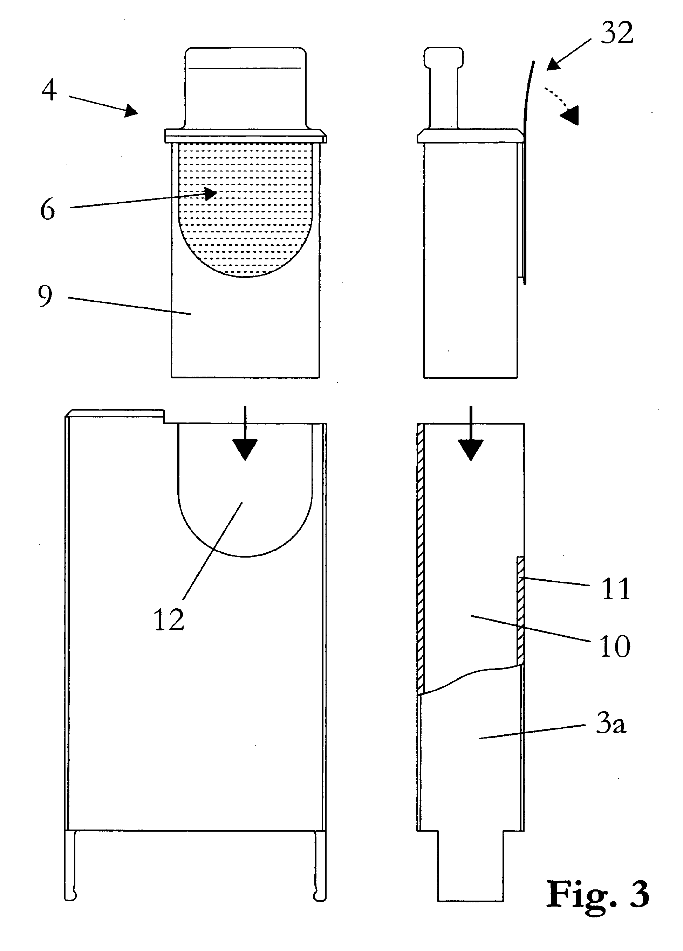

US 7,100,618 (Armando Dominguez) beschreibt einen sensorischen Rauchsimulator, bestehend aus einem Gehäuse 40, welches zwei durch eine Trennwand 43 getrennte Kammern 41, 42 beinhaltet. Die untere Kammer 42 weist auf einer Seite ein Luft-Einlaßfenster 10 auf. Auf der gegenüberliegenden Seite ist sie mit einem rohrförmigen Mundstückhalter 14a verbunden. Der rohrförmige Mundstückhalter 14a hält ein Mundstück 15 mit einem inneren Durchgang 14b. Das Mundstück 15 weist ähnlich wie Zigarettenrauch einen bitteren Geschmack auf. In der unteren Kammer 42 sind zwei Flügelräder 12 angeordnet, welche durch die vom Benutzer im Zuge einer Inhalation bewirkte Saugströmung in Rotation versetzt werden. Die Flügelräder 12 treiben ihrerseits über Wellen 16 zwei korrespondierende, in der oberen Kammer 41 angeordnete Flügelräder 29 an, welche wie eine Saugpumpe wirken. Die obere Kammer 41 weist auf einer Seite einen Zigarettenhalter 24 auf. Auf der gegenüberliegenden Seite sind zwei Auslaßröhrchen 30 mit der Kammer 41 verbunden und so angeordnet, daß deren Öffnungen während der Benutzung des Rauchsimulators in der Nähe der Nase des Benutzers zu liegen kommen. Die obere Kammer 41 ist durch einen Deckel 34 verschlossen. In den Deckel 34 ist eine Haupt-Auslaßöffnung 35 integriert. Zur Funktion des Rauchsimulators: der Benutzer setzt in den Zigarettenhalter 24 eine Zigarette 21a ein und zündet sie an. Sobald er über das Mundstück 15 eine Inhalation ausführt, setzen sich die Flügelräder 12 in Bewegung und treiben ihrerseits die Saugpumpenräder 29 an. Letztere bewirken, daß Luft aus der Umgebung durch die Zigarette 21a gesaugt wird, und sich im Zuge dessen wie beim herkömmlichen Rauchen Zigarettenrauch bildet, welcher Rauch durch die Kammer 41 gepumpt wird. Der überwiegende Teil des Rauches wird über die Haupt-Auslaßöffnung 35 ungenutzt wieder in die Umgebung ausgeblasen, während eine kleinere Menge des Rauches über die Auslaßröhrchen 30 gezielt in die Nähe der Nase des Benutzers ausströmt und von diesem als Tabakrauch-Aroma wahrgenommen werden kann. In einer alternativen Ausgestaltung des Rauchsimulators kann statt der Zigarette 21a auch ein mit Zigarettenrauch-Aroma imprägnierter Körper 21e in den Zigarettenhalter 24 eingesetzt werden (Fig. 3A). In einer weiteren alternativen Ausgestaltung kann statt der Flügelräder 12 auch ein elektrischer Motor 17 die Saugpumpenräder 29 antreiben (Fig. 2).

Die Trägheit der vier Flügelräder 12, 29, die Reibung in deren Lagern, die Spalte zwischen den Flügelrädern und dem Gehäuse 40 und das in den Kammern 41, 42 stets vorhandene Totvolumen haben zur Folge, daß das beim Zigarettenrauchen während eines Zuges üblicherweise in die Mundhöhle gesaugte Gasvolumen (ca. 20-80mL) wohl nicht ausreichen wird, um eine Funktion, wie zuvor beschrieben, zu bewirken. Dessen ungeachtet, weist der Rauchsimulator einen ziemlich komplizierten und aufwendigen Aufbau auf. Der Bauaufwand wird auch nicht reduziert, wenn die Flügelräder 12 durch einen Elektromotor 17 substituiert werden. Bei Verwendung der Zigarette 21a werden erhebliche Mengen gesundheitsschädlichen Nebenstromrauches gebildet und in die Umgebung freigesetzt. Über die Inhaltsstoffe des mit "Zigarettenrauch-Aroma" imprägnierten Körpers 21e werden keine näheren Angaben gemacht. JP 11-009693 (Nagai Kenichi) zeigt eine vom Prinzip her ähnliche Anordnung.

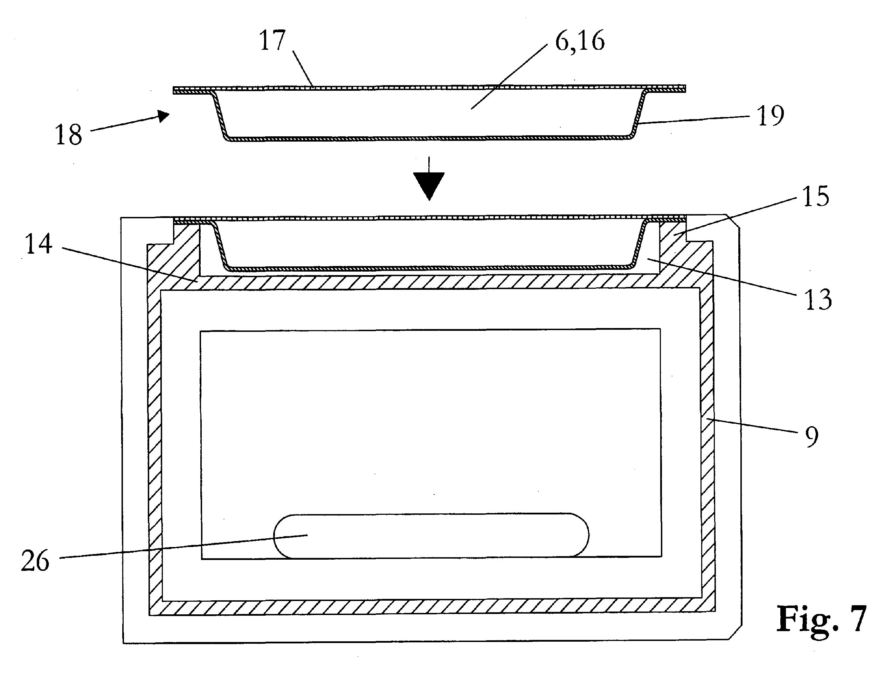

JP 08-056640 (Setsuo Kuroki) beschreibt ein Inhalationsgerät, im Wesentlichen bestehend aus (Fig. 3, 4 und 6) einer Trommel 7, beinhaltend einen Parfüm-imprägnierten Körper 6 oder einen Tabakrauch-Parfüm-imprägnierten Körper 6a, und ferner bestehend aus einer Mundstück-Einheit 10 mit einem Mundstück 11. Im vorderen Teil des Gerätes befindet sich ein erstes Rückschlagventil B, welches eine Eintrittsöffnung 3 öffnet und schließt. Im rückwärtigen Teil des Gerätes befindet sich ein zweites Rückschlagventil C, welches eine Austrittsöffnung 15 öffnet und schließt. Die Funktion des Gerätes ist in Fig. 7 dargestellt: während eines Zuges bzw. einer Inhalation strömt Luft aus der Umgebung über die Eintrittsöffnung 3 in das Gerät ein, durchsetzt den Parfüm-imprägnierten Körper 6 bzw. den Tabakrauch-Parfüm-imprägnierten Körper 6a, reichert sich mit Parfüm an und gelangt schließlich über das Mundstück 11 in die Mundhöhle des Benutzers. Das auf diese Weise verabreichte Luft-Parfüm-Gemisch wird nach der Inhalation nicht wie bei Zigaretten üblich in die Umgebung ausgeblasen, sondern über das Mundstück 11 in das Inhalationsgerät zurückgeblasen, wodurch im Gerät ein Überdruck entsteht. Der Überdruck führt dazu, daß sich das Rückschlagventil C öffnet, und das Luft-Parfüm-Gemisch durch die Austrittsöffnung 15 in Richtung der Nase des Benutzers ausströmt, wodurch schließlich das Luft-Parfüm-Gemisch vom Benutzer olfaktorisch wahrgenommen werden kann. Ein Zigarettenraucher müßte also sein Zug- bzw. Inhalationsverhalten komplett umstellen, wenn er dieses Inhalationsgerät benützen wollte. Dieser Umstand ist als Nachteil zu werten. Es gilt ferner als nachteilig, daß die olfaktorische Reizstimulierung nicht zugsynchron erfolgt. Über die Inhaltsstoffe des verabreichten Parfüms bzw. "Tabakrauch-Parfüms" werden keine näheren Angaben gemacht. JP 01-094861 (Watabe Isamu) zeigt eine vom Prinzip her ähnlich wirkende Anordnung, welche ohne die Verwendung von Rückschlagventilen auskommt.

US 2,809,634 (Hirotada Murai) und GB 408,856 (Wietske van Seters-Bosch et al.) beschreiben Inhalationsapparate, welche die gleichzeitige Inhalation eines inhalierbaren Mediums durch Mund und Nase ermöglichen. Bei der Apparatur nach US 2,809,634 handelt es sich um eine sehr aufwendige Konstruktion, bei welcher das inhalierbare Medium nach Betätigung eines Ventilmechanismus vom Benutzer bezogen werden kann, wobei die Betätigung des Ventilmechanismus durch das Zusammenbeißen der Zähne erfolgt. Im Fall der GB 408,856 muß der Benutzer außer einem Mundstück zusätzlich zwei Nasenschläuche applizieren. Viele Benutzer würden diese Apparaturen alleine schon wegen deren eigenartigen und beschränkt gesellschaftsfähigen Handhabung und Bedienung ablehnen. Nach der US 2,809,634 kann das inhalierbare Medium ein in Alkohol gelöstes Tabakextrakt oder Tabakrauch-Extrakt beinhalten. Als Lösungsmittel für die Extraktion wird beispielhaft Ether angeführt.

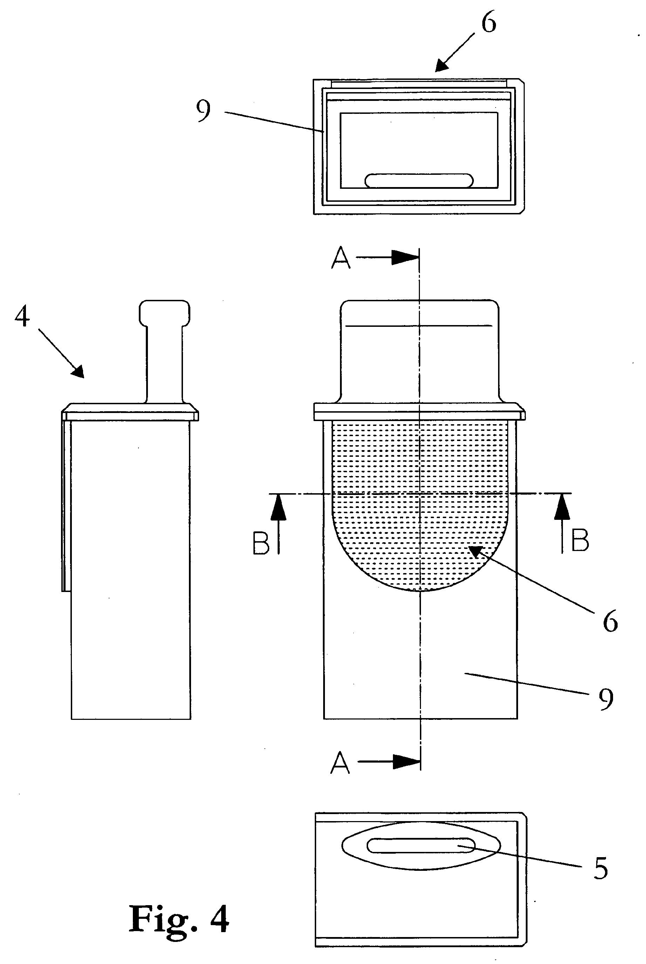

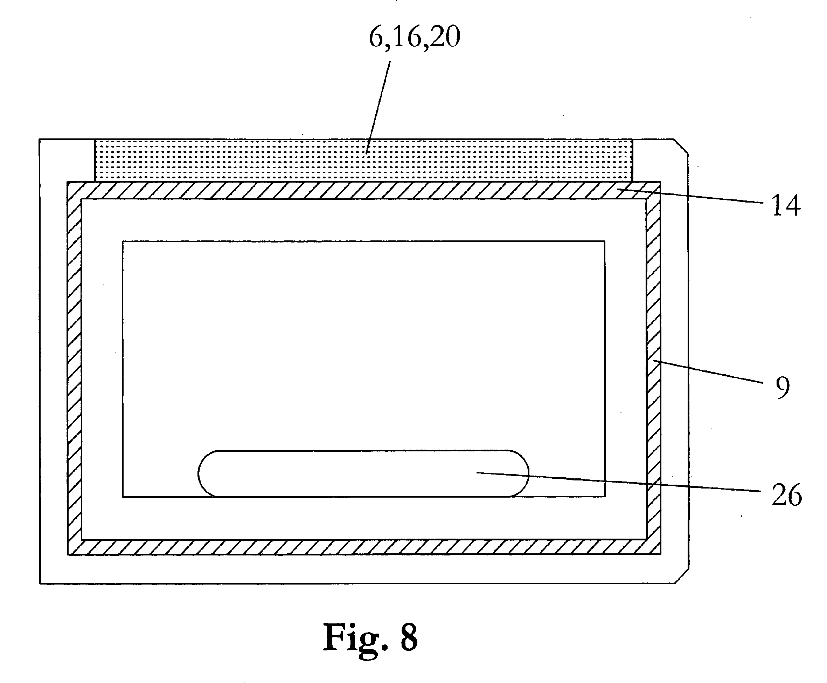

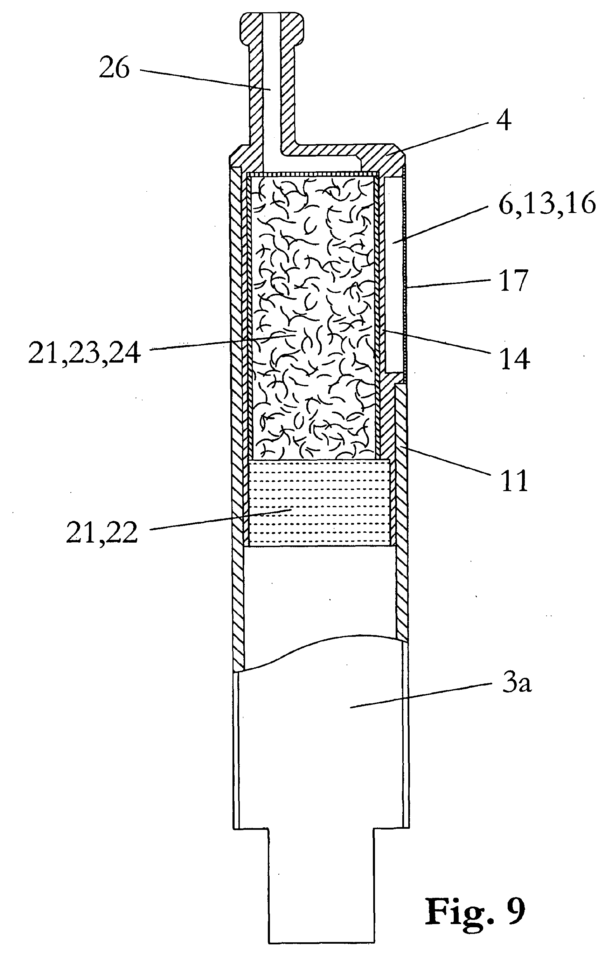

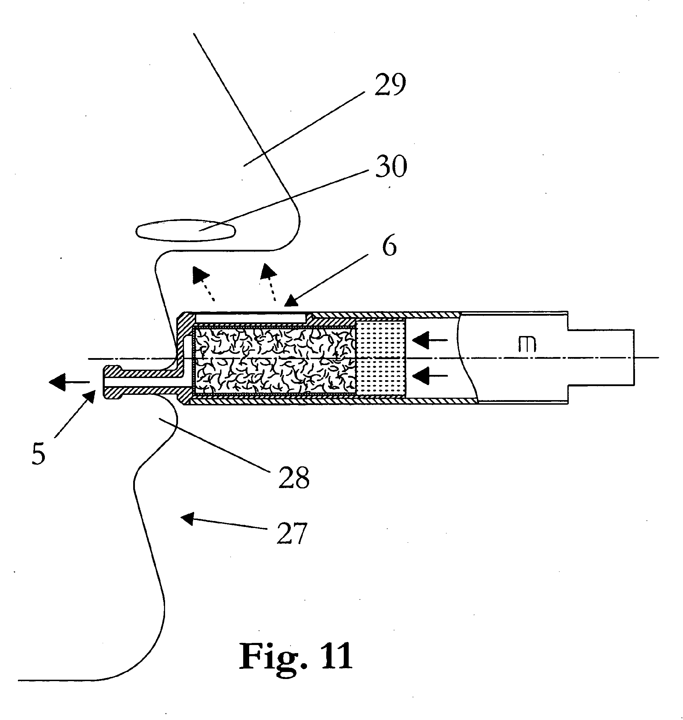

WO 88/01884 (Paul Terasaki) beschreibt verschiedene Varianten eines "Schnüffelstabs", welche allesamt umfassen: ein Mundstück 1, eine Verlängerung 2 und eine Duftstoff-Zubereitung 3, welche von der Verlängerung 2 gestützt wird. Wenn ein Benutzer das Mundstück 1 zwischen seinen Lippen oder Zähnen hält, befindet sich die Duftstoff-Zubereitung 3 in der Nähe seiner Nasenlöcher, wodurch die Duftstoffe in effizienter Weise konsumiert werden können. Im Ausführungsbeispiel nach Fig. 4 bildet ein einzelner Kunststoffstab sowohl das Mundstück 1 als auch die Verlängerung 2 aus. Die Duftstoff-Zubereitung 3 ist in einem Papier-Substrat 4 absorbiert, welches um die Verlängerung 2 gewickelt ist und mittels eines Klebstoffes am Kunststoffstab fixiert ist. Fig. 7 zeigt vom Prinzip her eine ähnliche Anordnung; der wesentliche Unterschied besteht darin, daß im Ausführungsbeispiel nach Fig. 7 sowohl das Mundstück 1 als auch die Verlängerung 2 flach ausgebildet sind. Fig. 8 und 9 zeigen Anordnungen mit abgewinkelter Verlängerung 2. Diese Varianten haben den Effekt, daß das Duftstoff-getränkte Substrat 4 während des Gebrauchs noch näher an die Nasenlöcher des Benutzers heranrückt. Fig. 11-37 zeigen Anordnungen mit stabförmigen Substraten 4, welche in von zylindrischen Verlängerungen 2 ausgebildeten Hohlräumen 8 angeordnet sind. Es sind verschiedenartige Steuereinrichtungen vorgesehen, mittels welcher der Benutzer auf die Freisetzung der Duftstoffe in gewissen Grenzen Einfluß nehmen kann. Die WO 88/01884 offenbart einen "Schnüffelstab" für die ausschließliche Verabreichung von Duftstoffen in diese Nase eines Benutzers, nimmt jedoch keinerlei Bezug auf Inhalatoren und kann folglich dem Fachmann auch keine konkrete Lehre vermitteln, wie ein Duftstoff-Substrat vorteilhaft in eine Inhalator-Anordnung integriert werden könnte.

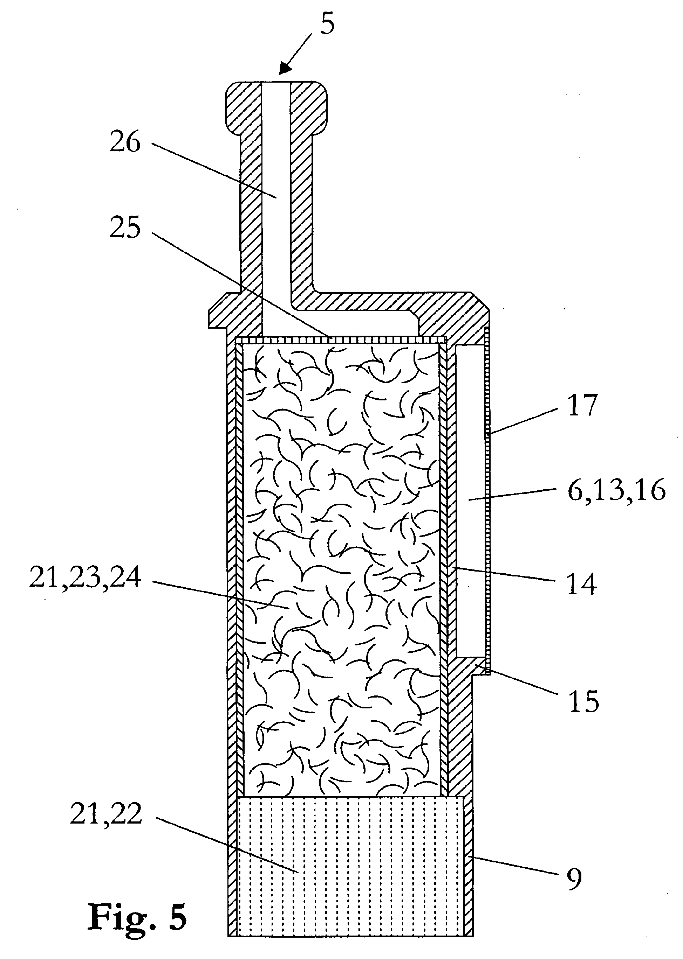

US 4,503,851 (Klaus Braunroth) beschreibt eine Einweg-Atemschutzmaske zur Maskierung von unangenehmen Gerüchen für die Verwendung in einem übelriechenden Umfeld, beispielsweise im Umfeld von Tierabfällen. Die Atemschutzmaske 40 basiert auf (Fig. 5-7) einer luftduchlässigen, filternden Gewebeabdeckung 41, welche mittels Bändern 42 am Kopf gesichert werden kann. Etwa in der Mitte der Gewebeabdeckung 41 befindet sich ein geruchsmaskierendes Mittel 50, bestehend aus einer undurchlässigen Hülle 52. Die Hülle 52 setzt sich aus zwei einander gegenüberliegenden Flächen 52A und 52B zusammen. Zwischen den Flächen 52A und 52B befindet sich ein absorbierendes Material 54, zum Beispiel Baumwolle. Das absorbierende Material 54 ist mit einer geruchsmaskierenden Substanz 55 getränkt. Die Fläche 52B weist eine Vielzahl von Öffnungen 56 auf und ist mit einer Dichtung 60 belegt, welche am äußeren Umfang der Hülle 52 verklebt ist. Die Dichtung 60 weist eine Lasche 62 auf, mittels welcher sich die Dichtung 60 von der Hülle 52 entfernen läßt. Nach Entfernung der Dichtung 60 ist die Atemschutzmaske 40 einsatzbereit. Die geruchsmaskierende Substanz 55 kann nun durch die Öffnungen 56 verdunsten und sich mit der durch die Gewebeabdeckung 41 durchströmenden, geruchsbelasteten Luft mischen und jeglichen Geruch neutralisieren.

Abschließend sei angemerkt, daß der in den zuvor dargestellten Dokumenten offenbarte Stand der Technik formal nicht der eingangs bezeichneten Gattung zuzurechnen ist. Die Dokumente wurden dennoch angeführt, da sie zumindest das weitere Umfeld der der gegenständlichen Anmeldung zugrundeliegenden Erfindung abbilden und insofern würdig sind, berücksichtigt zu werden.

US 5 501 236 A offenbart eine Inhalator-Komponente, die aus einem Gehäuse und einem eine Mundstücköffnung aufweisenden Mundstück aufgebaut ist.

Der Erfindung liegt die Aufgabe zugrunde, die zuvor aufgezeigten Nachteile des Standes der Technik zu beheben. Der Erfindung liegt insbesondere die Aufgabe zugrunde, eine Inhalatorkomponente der eingangs geschilderten Art so auszugestalten, daß
-) der Riechstoff in effizienter Weise verabreicht wird, und unbeteiligte Dritte durch die Riechstoff-Freisetzung möglichst nicht belästigt werden;
-) die Gesamtanordnung der Inhalatorkomponente kompakt bleibt;
-) die Inhalatorkomponente einfach, ergonomisch und bedienerfreundlich zu handhaben ist, und ihr Gebrauch hygienisch ist;
-) die olfaktorischen Effekte des Zigaretten-Nebenstromrauches so realistisch wie möglich nachgebildet werden.

Die Aufgabe wird dadurch gelöst, daß
a) das Gehäuse eine Gehäusekomponente umfaßt;
b) das Mundstück mit der Gehäusekomponente trennbar verbunden ist;
c) der Gehäusemantel ein erstes Mantelteil und ein zweites Mantelteil umfaßt;
d) die Gehäusekomponente das erste Mantelteil ausbildet;
e) das Mundstück das zweite Mantelteil ausbildet, und insbesondere dadurch, daß
f) das Riechstoff-Reservoir mit dem Mundstück baulich vereinigt ist, flach, platten-oder schalenförmig ausgebildet ist und flächig am zweiten Mantelteil angeordnet ist oder selbst das zweite Mantelteil ausbildet.

Dementsprechend löst die vorliegende Erfindung die ihr zugrundeliegende Aufgabe durch den Gegenstand der unabhängigen Ansprüche 1 und 14.

Die Verwendung des Singulars "Riechstoff-Reservoir" soll das Vorhandensein von mehreren Riechstoff-Reservoiren nicht ausschließen. Es gelte ausdrücklich als erfindungsgemäß, daß auch mehrere Riechstoff-Reservoire vorhanden sind. Die flächige, zum Beispiel platten- oder schalenförmige Ausbildung des Riechstoff-Reservoirs und dessen flächige Anordung am Gehäusemantel bzw. Integration in den Gehäusemantel haben den Effekt, daß einerseits andere Funktionsteile innerhalb des Gehäusemantels, insbesondere Funktionsteile für die Bildung des inhalierbaren Mediums, in ihrer Anordnung nicht oder nicht wesentlich tangiert werden, und andererseits das Riechstoff-Reservoir stufenfrei oder annähernd stufenfrei an benachbarte Gehäuseabschnitte anschließt, was nicht nur aus ästhetischer Sicht als Vorteil zu werten ist, sondern auch das Handling der Inhalatorkomponente vereinfacht. Praktische Ausführungsbeispiele für Riechstoff-Reservoire, insbesondere für plattenförmige Riechstoff-Reservoire werden später noch dargestellt.

Durch die Anordnung des Riechstoff-Reservoirs am vom Mundstück ausgebildeten zweiten Mantelteil bzw. durch die Ausbildung des zweiten Mantelteils durch das Riechstoff-Reservoir wird erzwungen, daß das Riechstoff-Reservoir während des Gebrauches der Inhalatorkomponente nahe der Nasenlöcher des Benutzers zu liegen kommt. Der unter diesen Bedingungen freigesetzte Riechstoff kann vom Benutzer schon bei vergleichsweise niedrigen Freisetzungsraten olfaktorisch wahrgenommen werden, wobei die Wahrnehmung durch die Nasenatmung und den dadurch zusätzlich induzierten konvektiven Stofftransport noch weiter unterstützt wird. Andere Personen in der näheren Umgebung werden vom freigesetzten Riechstoff wegen des um ein Vielfaches größeren Abstandes zum Riechstoff-Reservoir kaum tangiert. Durch die bauliche Vereinigung des Riechstoff-Reservoirs mit dem auswechselbaren Mundstück ist es möglich, beide Bauteile gemeinsam auszutauschen. Dies vereinfacht die Handhabung erheblich. Das Handling ist denkbar einfach: der Benutzer ergreift das freie Mundstückende und entkoppelt das Mundstück von der Gehäusekomponente. Mit dem Riechstoff-Reservoir kommt er dabei nicht in Berührung. Besonders günstig ist es, wenn das Riechstoff-Reservoir mit dem Mundstück untrennbar verbunden ist. Der Benutzer wird dadurch faktisch dazu gezwungen, das Mundstück regelmäßig durch ein neues Mundstück zu ersetzen, wenn er die Riechstoff-Freisetzung aufrechterhalten möchte. Der regelmäßige, zum Beispiel tägliche Austausch des Mundstücks ist aus hygienischer Sicht zweifelsohne vorteilhaft.

In einer vorteilhaften Weiterbildung der Erfindung ist vorgesehen, daß das erste Mantelteil und das zweite Mantelteil sich im Bereich des Riechstoff-Reservoirs oder/und in dessen Umfeld zumindest teilweise überlappen. In einer besonders günstigen Ausgestaltung der Erfindung ist ferner vorgesehen, daß das Riechstoff-Reservoir oder/und der das Riechstoff-Reservoir aufnehmende Abschnitt des zweiten Mantelteils zumindest teilweise in eine Ausparung oder Einbuchtung des ersten Mantelteils eingreift/eingreifen, oder das Riechstoff-Reservoir über eine Ausparung im ersten Mantelteil mit der Umgebung kommuniziert. Diese Anordnungen geben dem Konstrukteur bei der Positionierung des Riechstoff-Reservoirs am Gehäusemantel mehr Flexibilität und ermöglichen eine Verschachtelung mit anderen Funktionsbauteilen, wodurch die Inhalatorkomponente letztlich noch kompakter baut.

Erfindungsgemäß sind die Mundstücköffnung und das Riechstoff-Reservoir auf gegenüberliegenden Seiten einer Gehäuse-Mittelebene m angeordnet, wobei die Gehäuse-Mittelebene m definiert ist als eine Ebene, welche das Gehäuse in Mantelrichtung etwa in zwei gleich große Teile spaltet. Die Anordung hat den Effekt, daß das Riechstoff-Reservoir während des Gebrauches der Inhalatorkomponente noch näher an die Nasenlöcher des Benutzers heranrückt, wodurch der Riechstoff noch effizienter verabreicht werden kann, oder anders formuliert, die Freisetzungsrate bei gleicher olfaktorischer Wirkung weiter reduziert werden kann. Letztendlich kann auf diese Weise die Nutzungsdauer des Riechstoff-Reservoirs verlängert werden.

In einer Weiterbildung der Erfindung umfaßt das Mundstück außerdem einen von der Dampf- bzw. Partikelphase des inhalierbaren Mediums durchströmbaren Kühler; in einer anderen Weiterbildung umfaßt das Mundstück außerdem ein das inhalierbare Medium mit Aromastoffen anreicherndes Aromastoff-Reservoir. Das Mundstück wird bei diesen Weiterbildungen zu einer Multi-Funktionseinrichtung ausgebaut, welche neben dem Riechstoff-Reservoir auch noch einen Kühler oder/und ein Aromastoff-Reservoir beinhaltet. Alle Funktionseinrichtungen können gemeinsam durch eine einfache Manipulation ausgetauscht werden. Die Bedienung der Inhalatorkomponente vereinfacht sich dadurch erheblich. Der Kühler kühlt das inhalierbare Medium, wodurch es angenehmer zu konsumieren ist. Eine solche Kühlung hat sich insbesondere bei nikotinhaltigen Dampf-Luft-Gemischen und Kondensationsaerosolen als sehr vorteilhaft erwiesen, wenn diese in Zug-Inhalatoren durch Verdampfung von mittels Ethanol oder/und Wasser hochverdünnten Nikotinlösungen gebildet werden. Zug-Inhalatoren sind Inhalationsapparate, bei welchen das inhalierbare Medium dem Benutzer wie bei einer Zigarette in zwei Schritten zugeführt wird, nämlich zuerst als Zug in die Mundhöhle (erster Schritt) und - nach Absetzen des Inhalators - in Form einer daran anschließenden Lungeninhalation (zweiter Schritt). Der Kühler kann beispielsweise von einem durchströmbaren Porenkörper ausgebildet werden. Der Porenkörper kann zusätzlich aromatisiert werden. Im letzteren Fall wird das durchströmende Dampf-Luft-Gemisch bzw. Aerosol nicht nur gekühlt, sondern auch noch mit Aromastoffen angereichert, wodurch es noch angenehmer zu konsumieren ist. Es können selbstverständlich auch Aromastoff-Reservoire ohne oder ohne nennenswerte Kühlwirkung vorgesehen sein. So könnten beispielsweise die das inhalierbare Medium leitenden Kanalwände des Mundstücks mit einem Aromastoff beschichtet sein.

Besonders vorteilhaft ist es, wenn das Aromastoff-Reservoir als ein von der Umgebungsluft durchströmbares, aber von der Dampf- bzw. Partikelphase des inhalierbaren Mediums unbeaufschlagtes Reservoir ausgebildet ist. Der durch das Aromastoff-Reservoir strömende Luftstrom reichert sich mit dem Aromastoff an und vereinigt sich schließlich weiter stromabwärts außerhalb des Aromastoff-Reservoirs mit dem, die Dampf- bzw. Partikelphase transportierenden Hauptstrom des inhalierbaren Mediums. Durch diese Anordnung kann effektiv verhindert werden, daß sich im Aromastoff-Reservoir Kondensat oder/und Aerosolpartikel abscheiden, welche Abscheidungen die Aromastoff-Freisetzung empfindlich stören könnten.

Nach der Erfindung ist vorgesehen, daß der Riechstoff des Riechstoff-Reservoirs Tabak enthält. Als Tabak eignen sich insbesondere getrockneter fermentierter Tabak, rekonstituierter Tabak, expandierter Tabak oder Mischungen derselben. Da es sich bei Tabak um einen natürlichen Rohstoff handelt, kann wohl davon ausgegangen werden, daß erfindungsgemäße mit tabakhaltigen Riechstoff-Reservoiren ausgestattete Inhalatorkomponenten oder Inhalatoren bei vielen Benutzeren auf eine hohe Akzeptanz stoßen werden. Besonders vorteilhaft ist es, wenn der Tabak als Schnitttabak, vorzugsweise Feinschnitttabak, oder als feines Granulat oder Tabakmehl vorliegt. Die große Oberfläche dieser Tabakformen unterstützt die Freisetzung der im Tabak enthaltenen Riechstoffe.

Nach der Erfindung ist alternativ vorgesehen, daß der Riechstoff des Riechstoff-Reservoirs ein Tabakrauch-Kondensat oder ein Tabakextrakt oder eine flüchtige aromatische Fraktion der vorgenannten Stoffgemische enthält, der Riechstoff jedoch im Wesentlichen nikotinfrei ist. Nikotin selbst besitzt an sich durchaus verwertbare Riechstoff-Eigenschaften, welche jedoch in keinem Verhältnis zu seinen toxischen Wirkungen (LD50: 0,88 mg·kg⁻¹) stehen. Da das Riechstoff-Reservoir von außen zugänglich ist, wird der Nikotingehalt des Riechstoffes erfindungsgemäß begrenzt und zwar mit 0,5 Massen-%. Dieser Restnikotin-Gehalt gelte im Rahmen der vorliegenden Patentanmeldung noch als "nikotinfrei".

Unter dem Begriff "Tabakrauch-Kondensat" wird jeder niedergeschlagene Tabakrauch verstanden. Tabakrauch-Kondensat hat eine zähflüssige bis pastöse Konsistenz und eine gelblich-braune Farbe. Durch die Verwendung desselben als Riechstoff in einem erfindungsgemäßen Riechstoff-Reservoir gelingt es, wesentliche olfaktorische Effekte des Zigaretten-Nebenstromrauches nachzubilden. Die Gewinnung von Tabakrauch-Kondensat erfolgte bislang vornehmlich für analytische Zwecke im Labormaßstab im Zuge des maschinellen Abrauchens von Zigaretten und ähnlichen Rauchwaren. Die hierbei eingesetzten Verfahren zur Abscheidung des Kondensats lassen sich auf vier Prinzipien zurückführen:
1) Abscheidung durch Prallwirkung, zum Beispiel auf Filtern oder mittels Kapillarpresse ("Jet Impaction Trap");
2) Abscheidung mittels Kühlfalle;
3) Abscheidung im elektrischen Feld;
4) Abscheidung in geeigneten Lösungsmitteln, zum Beispiel in Waschflaschen, oder Niederschlagung mittels versprühter Lösungsmittel. Als Lösungsmittel kommen vor allem Wasser, Aceton und Ethanol in Betracht.

Ein großtechnisch-kommerziell angewandtes Verfahren für die Gewinnung größerer Mengen von Tabakrauch-Kondensat ist dem Anmelder nicht bekannt. Es existieren jedoch großtechnische Anlagen zur Gewinnung von Flüssigrauch-Produkten aus Holz für die Verwendung in oder auf Lebensmitteln als Ersatz für das Räuchern. Ein Beispiel für einen Betreiber von solchen Anlagen ist die Firma Red Arrow International LLC, www.redarrowinternational.com. Die Anlagen basieren auf der Pyrolyse von Hartholz-Sägemehl. Das Sägemehl wird unter weitgehendem Ausschluss von Sauerstoff erhitzt, und die entstehenden Rauchgase in Lösungsmitteln, wie Wasser, aufgefangen. Die großtechnische Gewinnung von Tabakrauch-Kondensat kann in analogen Anlagen erfolgen. Das zu pyrolysierende Ausgangsmaterial besteht aus getrocknetem fermentierten Tabak, wobei Tabak aus nikotinfreien Züchtungen oder entnikotinisierter Tabak den Vorteil haben, daß ohne zusätzliche Verfahrensschritte ein nikotinfreies Tabakrauch-Kondensat gewonnen werden kann.

Tabakrauch-Kondensate bestehen aus einer hochkomplexen Mischung von mehreren Tausenden Einzelsubstanzen; viele davon haben aromatische Eigenschaften und leisten insofern einen Beitrag zum Gesamtaroma des Tabakrauch-Kondensats; andere haben keine oder gar nachteilige Riechstoff-Eigenschaften. Die Flüchtigkeit aller dieser Inhaltsstoffe ist sehr unterschiedlich, was dazu führt, daß das Riechstoff-Reservoir in der Anfangsphase seiner Nutzung überdurchschnittlich große Mengen an leicht flüchtigen Substanzen freisetzt, wodurch sich das Riechstoff-Aroma über die Nutzungsdauer qualitativ verschiebt und in seiner Wirkung bald nachläßt. Durch eine Raffination kann gezielt auf die Inhaltsstoffe und Eigenschaften des Tabakrauch-Kondensats Einfluß genommen werden, insbesondere Inhaltsstoffe oder Fraktionen ohne oder mit nachteiligen Riechstoff-Eigenschaften ausgeschieden, das Flüchtigkeitsspektrum eingeengt, und günstige aromatische Komponenten aufkonzentriert werden. Die hierfür einzusetzenden Verfahren, wie Extraktion und Destillation, sind dem Fachmann der Riechstoff- und Aroma-Chemie geläufig. Das Endprodukt der Raffination ist eine nikotinfreie, flüchtige, konzentrierte aromatische Fraktion des Tabakrauch-Kondensats, oder mehrere solcher Fraktionen, beispielsweise Fraktionen mit unterschiedlichem Flüchtigkeitsspektrum.

Unter dem Begriff "Tabakextrakt" wird ein aus fermentiertem Tabak gewonnenes ätherisches Öl verstanden. Oft bezeichnet man diese Öle auch als "Tabakaromaöle". Die Gewinnung von nikotinfreien Tabakaromaölen kann beispielsweise nach Verfahren erfolgen, wie sie in den Patent-Publikationen DE 19654945 und DE 19630619 (Adam Müller et al.) detailliert dargestellt sind. Das Tabakaromaöl kann allenfalls noch in Fraktionen mit unterschiedlichem Flüchtigkeitsspektrum zerlegt werden.

Des Weiteren wird als erfindungsgemäß angesehen, daß der Riechstoff des Riechstoff-Reservoirs wenigstens eine flüchtige Säure enthält und der Anteil aller flüchtigen Säuren im Riechstoff in Summe größer als 5 Massen-% ist. Durch die flüchtige Säure soll dem Benutzer ein Geruchseindruck vermittelt werden, welcher dem leicht beißend-stechenden Geruch von Zigaretten-Nebenstromrauch ähnlich ist, verbunden mit einer vergleichbaren Irritation der Nasenschleimhaut. Als flüchtige Säuren kommen beispielhaft Essigsäure, Ameisensäure und Propionsäure, sowie Mischungen derselben in Betracht. Die Erfindung soll jedoch nicht auf diese Säuren beschränkt sein.

Schließlich ist nach der Erfindung vorgesehen, daß der Riechstoff des Riechstoff-Reservoirs Menthol enthält. Menthol hat eine erfrischende Wirkung. Seine Zugabe rundet das Gesamtaroma des Riechstoffes in angenehmer Weise ab. Das Menthol kann als Einzelsubstanz oder als Bestandteil eines ätherischen Öles, z.B. Pfefferminzöl, zugefügt werden.

Zweckmäßige und vorteilhafte Ausführungsbeispiele der Erfindung sind in den Zeichnungen dargestellt und werden in der nachfolgenden Beschreibung näher erläutert.

Es zeigen:
Fig.1 einen erfindungsgemäßen Inhalator in verschiedenen Ansichten;
Fig. 2 einen Inhalator nach Fig. 1 mit einem wiederverwendbaren Inhalatorteil und einer auswechselbaren Inhalatorkomponente im entkoppelten Zustand;
Fig. 3 die auswechselbare Inhalatorkomponente mit einem auswechselbaren Mundstück im entkoppelten Zustand in verschiedenen Ansichten;
Fig. 4 das auswechselbare Mundstück in verschiedenen Ansichten;
Fig. 5 eine Schnittansicht des auswechselbaren Mundstückes längs der Linie A-A in
Fig. 4;

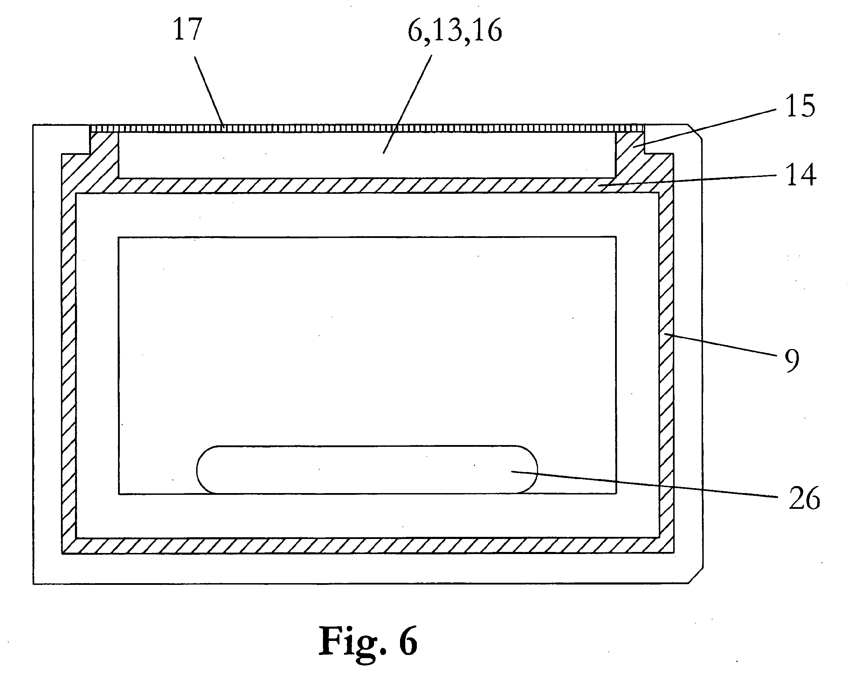

Fig. 6-8 jeweils eine Schnittansicht des auswechselbaren Mundstückes längs der Linie B-B in Fig. 4; verschiedene Riechstoff-Reservoir-Typen;
Fig. 9 eine Teilschnittansicht der auswechselbaren Inhalatorkomponente mit angekoppeltem Mundstück;

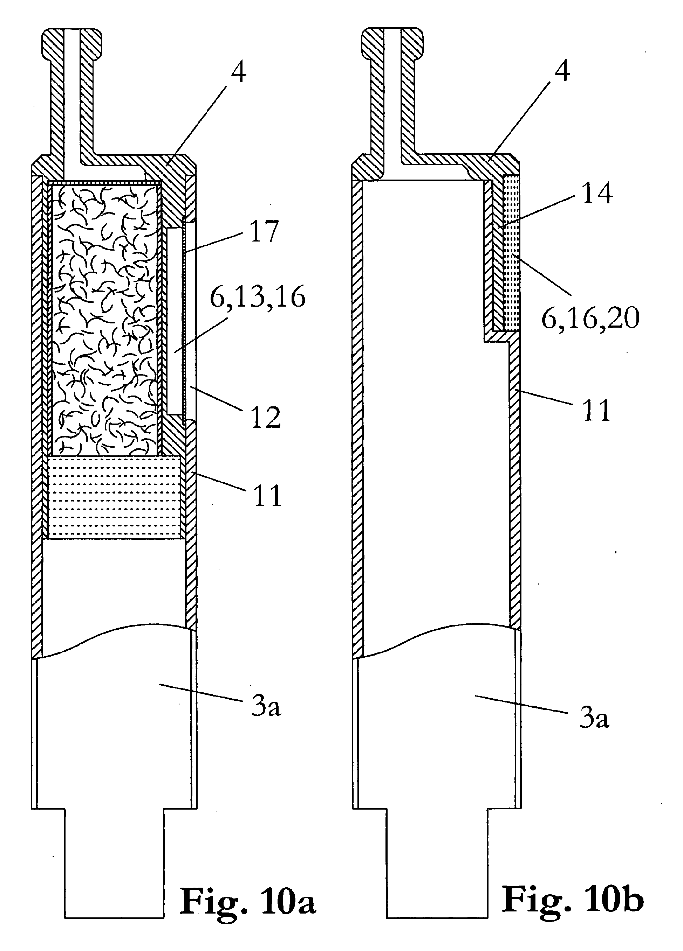

Fig. 10a und 10b jeweils eine Teilschnittansicht der auswechselbaren Inhalatorkomponente mit angekoppeltem Mundstück; zwei alternative Ausführungsvarianten;
Fig. 11 die auswechselbare Inhalatorkomponente gemäß Fig. 9 in Gebrauchsstellung;

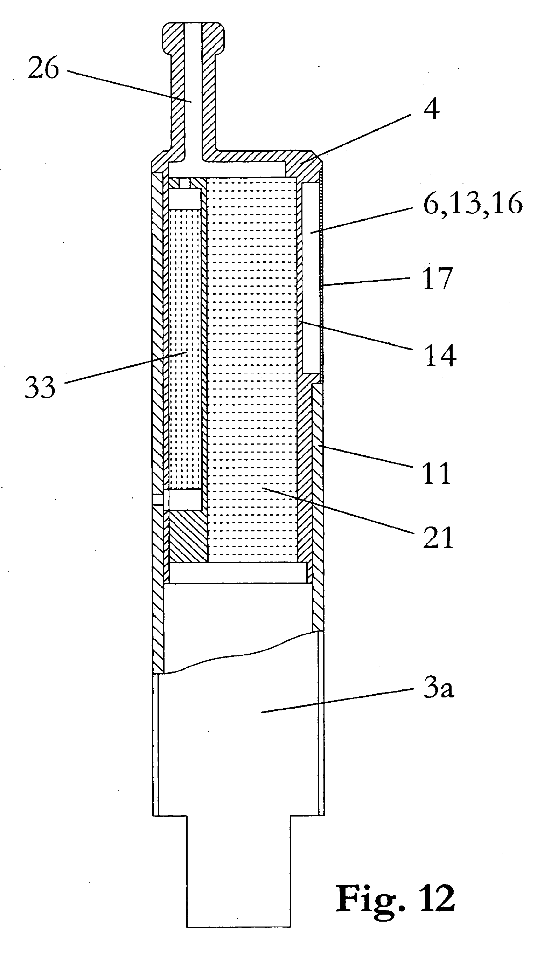

Fig. 12 eine Teilschnittansicht der auswechselbaren Inhalatorkomponente mit angekoppeltem Mundstück in einer weitergebildeten Ausführungsvariante;

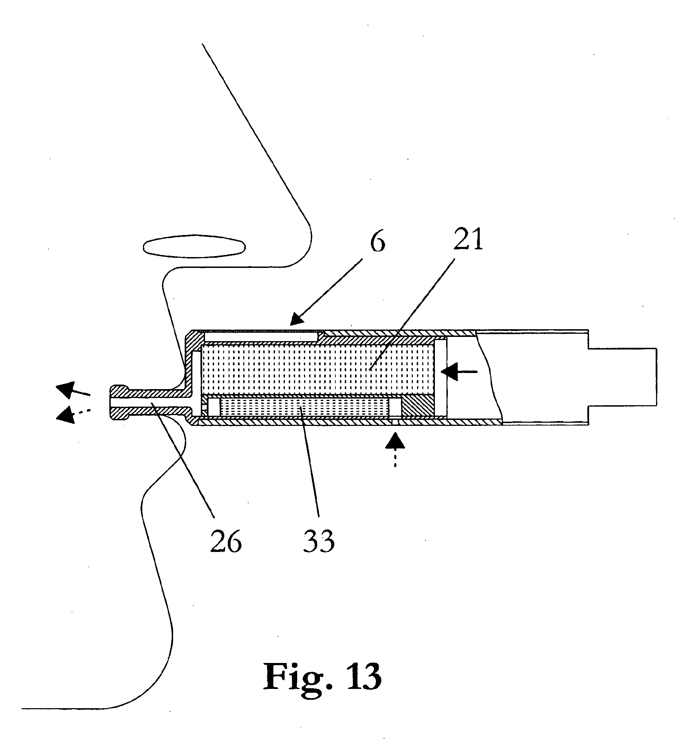

Fig. 13 die auswechselbare Inhalatorkomponente gemäß Fig. 12 in Gebrauchsstellung.

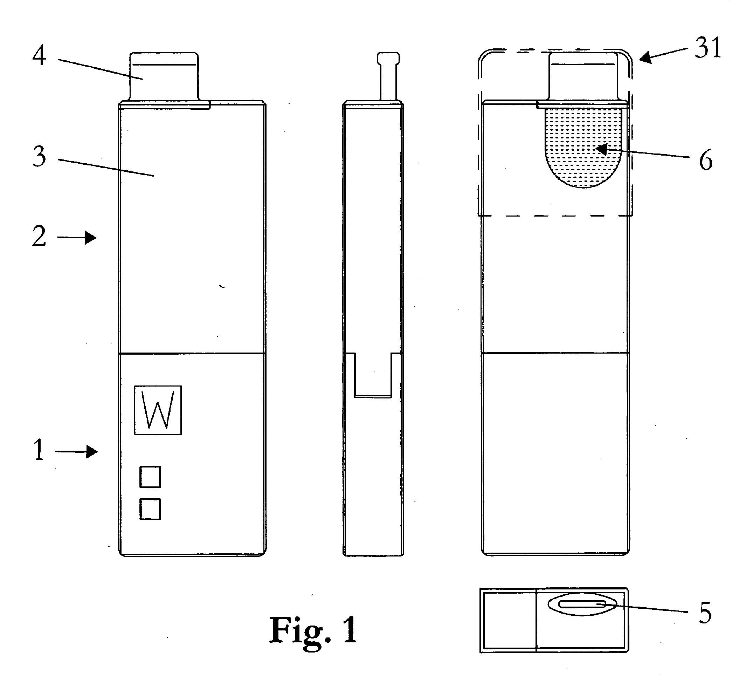

Fig. 1 zeigt einen beispielhaften erfindungsgemäßen Inhalator, welcher im konkreten Fall aus zwei Teilen besteht, nämlich aus einem Inhalatorteil 1 und einer Inhalatorkomponente 2. Die Inhalatorkomponente 2 besteht aus einem quaderförmigen Gehäuse 3. Die Inhalatorkomponente 2 umfaßt ferner ein Mundstück 4 mit einem tabakpfeifenartigen Mundstückende und einer Mundstücköffnung 5 für die Zufuhr eines inhalierbaren Mediums in die Mundhöhle eines Benutzers. Schließlich beinhaltet die Inhalatorkomponente 2 noch ein U-förmiges Riechstoff-Reservoir 6. Das Riechstoff-Reservoir 6 ist in den Gehäusemantel integriert, wie später noch genauer dargestellt wird. Das Gehäuse 3 und das Mundstück 4 sind vorzugsweise aus Kunststoff gefertigt.

Wie die Fig. 2 zeigt, sind das Inhalatorteil 1 und die Inhalatorkomponente 2 im vorliegenden Ausführungsbeispiel voneinander lösbar ausgeführt. Die lösbare Kopplung besteht aus einer Schnappverbindung, gebildet aus zwei Schnapphaken 7 und zwei mit diesen zusammenwirkenden Rastnasen 8. Diese Anordnung macht das Inhalatorteil 1 wiederverwendbar bzw. die Inhalatorkomponente 2 austauschbar und ist vor allem dann vorteilhaft einsetzbar, wenn das Inhalatorteil 1 Bestandteile enthält, welche langlebiger sind als die Bestandteile der Inhalatorkomponente 2. Solche langlebigeren Bestandteile können beispielsweise wiederaufladbare Batterien sowie elektronische Schaltkreise sein. Betont werden muß, daß die Erfindung keinesfalls auf dieses zweiteilige Inhalatorkonzept beschränkt ist. Die Erfindung ist vielmehr auf jeden Inhalatortyp anwendbar, sofern dieser ein Mundstück für die Zufuhr eines inhalierbaren Mediums in die Mundhöhle eines Benutzers aufweist. So könnten das Inhalatorteil 1 und die Inhalatorkomponente 2 selbstverständlich auch einteilig, also voneinander untrennbar ausgeführt sein, wobei in diesem Fall der ganze Inhalator als Inhalatorkomponente 2 aufzufassen wäre.

Wie die Fig. 3 zeigt, ist das Mundstück 4 mit einer Gehäusekomponente 3a trennbar verbunden. Die Gehäusekomponente 3a und Teile des Mundstückes 4 bilden zusammen das quaderförmige Gehäuse 3. Das Mundstück 4 bildet auf der der Mundstücköffnung 5 abgewandten Seite einen Hohlzylinder 9 aus, auf dessen Mantel seinerseits das Riechstoff-Reservoir 6 angeordnet ist, wie nachfolgend noch genauer ausgeführt wird. Das Riechstoff-Reservoir 6 kann also gemeinsam mit dem Mundstück 4 ausgewechselt werden. Die Kopplung des Mundstückes 4 mit der Gehäusekomponente 3a erfolgt über den Hohlzylinder 9, indem derselbige in eine korrespondierende Öffnung 10 der Gehäusekomponente 3a eingeschoben wird. Die Gehäusekomponente 3a bildet ferner eine Mantelkomponente 11 aus. In der Mantelkomponente 11 ist eine U-förmige Aussparung 12 vorgesehen, welche im Zuge der Kopplung des Mundstückes 4 dazu dient, das U-förmige Riechstoff-Reservoir 6 passgenau aufzunehmen, so daß das Riechstoff-Reservoir 6 frei mit der Umgebung kommunizieren kann.

Die Fig. 4-6 zeigen das auswechselbare Mundstück 4 für sich allein nochmals im Detail. Fig. 9 zeigt das Mundstück 4 mit der Gehäusekomponente 3a gekoppelt. Wie den Figuren zu entnehmen ist, weist der Hohlzylinder 9 einen rechteckigen Querschnitt auf. Das U-förmige Riechstoff-Reservoir 6 besteht im vorliegenden Ausführungsbeispiel aus einer flachen Ausnehmung 13 im Zylindermantel 14 des Hohlzylinders 9. Die Ausnehmung 13 wird von einer Erhebung 15 berandet. Die Erhebung 15 wird vom Zylindermantel 14 gebildet. Im Zuge der Kopplung des Mundstückes 4 mit der Gehäusekomponente 3a greifen das Riechstoff-Reservoir 6 und die das Riechstoff-Reservoir 6 einfassende Erhebung 15 in die Aussparung 12 der Mantelkomponente 11 ein (siehe Fig. 9).

Im Riechstoff-Reservoir 6, das heißt in der Ausnehmung 13, befindet sich der eigentliche Riechstoff 16, welcher in flüssiger, pastöser, fester oder gelartiger Form vorliegen kann. Das Riechstoff-Reservoir 6 ist zur Umgebung hin durch eine gasdurchlässige Membran 17 begrenzt. Die Membran 17 wird an der Erhebung 15 durch Verschweißen oder Verschmelzen befestigt, und dient mehreren Zwecken: Erstens verhindert sie ein Auslaufen des Riechstoffes 16 aus dem Reservoir 6. Zweitens drosselt bzw. steuert sie die Riechstoff-Freisetzung in die Umgebung. Drittens verhindert sie das Eindringen von Feuchtigkeit, insbesondere von Regenwasser, in das Riechstoff-Reservoir 6. Und schließlich verhindert sie, daß der Benutzer mit dem Riechstoff 16 in Kontakt kommt. Nicht alle der vorgenannten Wirkungen müssen zwingend in Erscheinung treten. So ist es zum Beispiel optional möglich, den Riechstoff 16 in einem saugfähigen, dochtartigen Porenkörper zu speichern, d.h. kapillar zu binden, wie später noch näher ausgeführt wird. In diesem Fall wäre die erstgenannte Wirkung der Membran 17 verzichtbar. Die gasdurchlässige Membran 17 kann beispielsweise aus einer thermoplastischen Kunststoffolie, zum Beispiel aus Polyethylen oder aus einem Ethylen-Vinylacetat-Copolymer, bestehen. Der flüchtige Riechstoff 16 diffundiert molekular durch die Folie hindurch. Mikroporöse thermoplastische Polymermaterialien erscheinen für die Zwecke der gegenständlichen Erfindung besonders geeignet. Die Firma Celgard LLC, www.celgard.com, bietet ein solches Material beispielsweise in Form von hydrophoben Polypropylen-Membranen an. Die Membranen werden unter der Handelsbezeichnung CELGARD® vertrieben. EP 836,857 (Eric Jallerat) sowie US 4,917,301 (Marina A. Munteanu) beschreiben alternative mikroporöse Polymermaterialien zum Zwecke der Freisetzung von Riechstoffen. Die Durchlässigkeit oder Permeabilität der Membran 17 ist auf die spezifischen Riechstoff-Eigenschaften (Geruchsschwelle, Flüchtigkeit) des im Reservoir 6 gespeicherten Riechstoffes 16 abzustimmen. Unter Umständen kann es vorteilhaft sein, mehrere voneinander unabhängige Riechstoff-Reservoire mit unterschiedlichen Membran-Eigenschaften vorzusehen, vor allem dann, wenn die Riechstoff-Eigenschaften der im Riechstoff enthaltenen Stoffe besonders weit streuen. Liegt der Riechstoff in fester Form vor, beispielsweise als Feinschnitttabak oder als feines Tabakgranulat, so kann die Membran 17 auch einfach nur aus einem feinmaschigen Drahtgewebe bestehen.

Fig. 7 zeigt eine alternative Ausgestaltung des Riechstoff-Reservoirs 6. Danach wird das Riechstoff-Reservoir 6 einschließlich der Membran 17 aus einer vorfabrizierten flachen Riechstoff-Packung 18 gebildet, welche in eine korrespondierende Ausnehmung 13 im Zylindermantel 14 des Mundstückes 4 einsetzbar ist, und an der Erhebung 15 mittels einer Klebeverbindung befestigt wird. Eine Riechstoff-Packung dieser Art wurde bereits früher im Zuge der Besprechung der US 4,503,851 (Klaus Braunroth) vorgestellt. In der Patentliteratur wurden solche Packungen erstmals in US 4,094,119 (William E. Sullivan), US 4,145,001 (Robert J. Weyenberg et al.) und US 4,161,283 (Sy Hyman) beschrieben. Die Riechstoff-Packung 18 besteht im Wesentlichen aus zwei Teilen: zum einen aus einer gasdurchlässigen Membran 17, wie zuvor beschrieben, und zum anderen aus einer impermeablen Folie 19, beispielsweise bestehend aus einem Kunststoff-Laminat oder aus einem Aluminium-Kunststoff-Laminat. Die impermeable Folie 19 ist mit der Membran 17 umfangsseitig verschweißt oder verschmolzen und bildet so im Inneren das Riechstoff-Reservoir 6 aus. Die Verwendung einer vorfabrizierten und befüllten Riechstoff-Packung 18 in einer erfindungsgemäßen Inhalatorkomponente 2 bzw. in einem erfindungsgemäßen auswechselbaren Mundstück 4 bietet Kostenvorteile bei der Massenherstellung derselben.

Fig. 8 zeigt eine weitere alternative Ausgestaltung des Riechstoff-Reservoirs 6. Das Riechstoff-Reservoir 6 wird in diesem Fall aus einem den Riechstoff 16 in seinen Poren aufnehmenden Porenkörper 20 gebildet. Der plattenförmige Porenkörper 20 besteht aus einem saugfähigen, dochtartigen Material und ist mittels einer Klebeverbindung am Zylindermantel 14 des Mundstückes 4 befestigt. Das Material des Porenkörpers 20 sollte möglichst formbeständig und gegenüber dem Riechstoff 16 weitgehend inert sein. Hierfür bieten sich an:
-) Faserformkörper aus thermisch oder mit Hilfe eines Bindemittels miteinander verbundenen Natur- oder Chemiefasern; die Firma Filtrona Richmond Inc., www.fltronaporoustechnologies.com, ist auf die Herstellung solcher Faserverbunde spezialisiert, wobei sowohl mittels Triacetin gebundene Celluloseacetat-Fasern als auch thermisch gebundene Polyolefin- und Polyesterfasern verarbeitet weden;
-) Offenporige Sinterformkörper, z.B. porös-gesintertes Polyethylen- oder Polypropylen- Material der Firma Porex Technologies, www.porex.com;
-) Schaummaterial, insbesondere Metallschaum; die Firma Mitsubishi Materials Corporation, www.mmc.co.jp, hat ein solches Schaummaterial in Edelstahlausführung (z.B. AISI 316L) in Dicken bis 2mm und mit einer Porosität >90% standardmäßig im Programm.

Pastöse und feste Riechstoffe können zuvor in einem geeigneten Lösungsmittel gelöst oder dispergiert werden, damit sie vom Porenkörper 20 leichter absorbiert werden können. Im Ausführungsbeispiel nach Fig. 8 kommuniziert das Riechstoff-Reservoir 6 bzw. der Porenkörper 20 unmittelbar mit der Umgebung. Eine Membran ist nicht vorgesehen. Eine solche einfache Anordnung bietet sich vor allem bei Verwendung von Riechstoffen mit geringer Flüchtigkeit oder/und hoher Geruchsschwelle an. Die Erfindung ist selbstverständlich nicht auf diesen Spezialfall beschränkt. Der Porenkörper 20 könnte vielmehr genauso in einer Ausnehmung 13 nach Fig. 6 angeordnet sein, oder in eine Riechstoff-Packung 18 nach Fig. 7 eingesetzt werden.

Wie Fig. 5 und Fig. 9 zeigen, befindet sich innerhalb des Hohlzylinders 9 ein vom inhalierbaren Medium durchströmbarer Kühler 21. Der Kühler nach dem vorliegenden Ausführungsbeispiel ist speziell für die Kühlung eines durch Verdampfung einer hochverdünnten Nikotinlösung gebildeten Dampf-LuftGemisches oder/und Kondensationsaerosols ausgelegt. Der Kühler 21 ist zweistufig aufgebaut und besteht aus einem Vorkühler 22 und einer diesem nachgeordneten Tabakfüllung 23. Der Vorkühler 22 bildet die erste Kühlerstufe und hat die Aufgabe, das Dampf-Luft-Gemisch bzw. Kondensationsaerosol möglichst effektiv und weitgehend vorzukühlen, bevor dieses die Tabakfüllung 23 erreicht. Der Vorkühler bewirkt, daß die Tabakfüllung 23 weniger mit Lösungsmittelkondensat beladen wird. Eine allzu große Kondensatbeladung der Tabakfüllung 23 kann die aromatisierende Wirkung derselben nachteilig beeinflussen. Der Vorkühler 22 kann beispielsweise aus einem Synthetikfaservlies bestehen. Die Firma Freudenberg Vliesstoffe KG, www.freudenberg-filter.com, bietet ein solches Material in Form von Matten/ Platten unter der Bezeichnung Viledon®-Filtermatten an. Das Material kann nach Kundenspezifikation angefertigt werden. Insbesondere können die Materialeigenschaften so abgestimmt werden, daß das Endprodukt für die feinen Partikel des erzeugten Kondensationsaerosols weitestgehend durchlässig ist. Die Matten/ Platten werden aus Polyolefinfasern (PE, PP) oder Polyesterfasern gefertigt und können durch Stanzen weiterverarbeitet werden. Die Tabakfüllung 23 liegt als vorfabrizierte Packung vor und besteht aus einem papierumwickelten Tabakstrang 24, welcher vorteilhafterweise aus einem Endlosstrang gewonnen wird. Der Tabakstrang 24 sowie der Vorkühler 22 sind in den Hohlzylinder 9 eingeschoben. Der Vorkühler 22 ist im Hohlzylinder 9 mittels einer Klebeverbindung befestigt, wodurch mittelbar auch der Tabakstrang 24 fixiert wird. Mundstückseitig wird die Tabakfüllung 23 durch ein Drahtgewebe 25 begrenzt, welches verhindert, daß das Füllmaterial in den Mundstückkanal 26 oder gar in die Mundhöhle des Benutzers gelangen kann. Der Vorkühler 22 und der Tabakstrang 24 bilden also mit dem Mundstück 4 eine bauliche Einheit, welche zusammen mit dem Riechstoff-Reservoir 6 auswechselbar ist. Der Tabakstrang 24 kann optional wie Zigaretten zusätzlich aromatisiert oder/und mentholisiert werden, wobei analoge Verfahren zur Anwendung kommen können.

Fig. 10a und Fig. 10b zeigen zwei alternative Ausgestaltungsbeispiele der Inhalatorkomponente 2 mit angekoppeltem Mundstück 4. Die Anordung nach Fig. 10a weist wie das zuvor dargestellte Ausführungsbeispiel (siehe Fig. 3 und Fig. 9) eine Aussparung 12 in der Mantelkomponente 11 auf. Das Riechstoff-Reservoir 6 greift hier jedoch nicht in die Aussparung 12 ein, sondern ist etwas nach innen versetzt angeordnet und kommuniziert über die Aussparung 12, quasi wie durch ein Fenster, mit der Umgebung. Das Ausgestaltungsbeispiel nach Fig. 10b zeigt eine Anordnung, bei welcher das Mundstück 4 ein Mantelteil 14 ausbildet, welches die Mantelkomponente 11 im Bereich des Riechstoff-Reservoirs 6 teilweise überlappt. Prinzipiell wäre auch eine selbsttragende Anordnung möglich, bei welcher das Riechstoff-Reservoir 6 selbst das Mantelteil 14 ausbildet und die Mantelkomponente 11 unmittelbar überlappt. Beide alternativen Ausgestaltungsbeispiele ermöglichen ebenfalls relativ kompakte Gesamtanordnungen.

Fig. 11 zeigt schließlich noch die Inhalatorkomponente 2 in ihrer Gebrauchsstellung. Die Strömung des inhalierbaren Mediums ist durch durchgezogene Pfeile symbolisiert; die Freisetzung des Riechstoffes ist durch strichlierte Pfeile gekennzeichnet. Wie deutlich zu erkennen ist, beeinflussen die beiden Stoffströme einander nicht. Das inhalierbare Medium besteht vorzugsweise aus einem nikotinhaltigen Dampf-Luft-Gemisch oder/und Aerosol. Die Herkunft des inhalierbaren Mediums bzw. die Art der Bildung desselben innerhalb der Inhalatorkomponente 2 ist für den Erfindungsgegenstand nicht von Relevanz und braucht daher in diesem Rahmen auch nicht näher dargestellt zu werden.

Ein Benutzer 27 hält das tabakpfeifenartige, flächig geformte Mundstückende des Mundstückes 4 zwischen seinen Lippen 28. Das flächige Mundstückende ist parallel zum plattenförmigen Riechstoff-Reservoir 6 ausgerichtet. Es wird dadurch erzwungen, daß das Riechstoff-Reservoir 6 stets genau gegenüber der Nase 29 bzw. gegenüber den Nasenlöchern 30 des Benutzers 27 positioniert wird. Ferner setzt das tabakpfeifenartige Mundstückende nicht zentrisch, sondern versetzt an der Stirnfläche des Gehäuses 3 an, so daß die Mundstücköffnung 5 und das Riechstoff-Reservoir 6 auf gegenüberliegenden Seiten der Gehäuse-Mittelebene m zu liegen kommen. Durch diese konstruktive Maßnahme rückt das Riechstoff-Reservoir 6 weitestmöglich an die Nasenlöcher 30 heran, ohne jedoch aus der Gehäuseoberfläche der Inhalatorkomponente 2 hinauszuragen, wodurch der Inhalator gut handhabbar bleibt und auch ästhetischen Ansprüchen gerecht wird. Der geringe Abstand des Riechstoff-Reservoirs 6 zu den Nasenlöchern 30 ermöglicht einen hohen Stofftransport-Wirkungsgrad, was bedeutet, daß ein vergleichsweise großer Teil der insgesamt freigesetzten Riechstoffmenge auch tatsächlich in die Nasenlöcher 30 gelangt und olfaktorisch zur Wirkung kommen kann.

Fig. 12 zeigt die auswechselbare Inhalatorkomponente in einer weitergebildeten Ausführungsvariante. Diese unterscheidet sich von der Ausführung gemäß Fig. 9 dadurch, daß neben dem Kühler 21 ein separates Aromastoff-Reservoir 33 vorgesehen ist. Das Aromastoff-Reservoir 33 bildet mit dem Kühler 21 und dem Mundstück 4 wieder eine bauliche Einheit, welche zusammen mit dem Riechstoff-Reservoir 6 auswechselbar ist. Wie Fig. 13 zeigt, erfolgen die Aromatisierung und Kühlung des inhalierbaren Mediums räumlich getrennt voneinnander, wobei zwei Stoffströme in Erscheinung treten: erstens eine Luftströmung durch das Aromastoff-Reservoir 33, welche aus der Umgebung gespeist wird (durch strichlierte Pfeile symbolisiert), und zweitens die eigentliche Hauptströmung durch den Kühler 21, welche dem Dampf-Luft-Gemisch bzw. Aerosol vorbehalten bleibt (durch durchgezogene Pfeile symbolisiert). Die beiden Stoffströme vereinigen sich weiter stromabwärts im Eingangsbereich zum Mundstückkanal 26 und gelangen schließlich gemeinsam in die Mundhöhle des Benutzers. Die weitgehende Trennung der beiden Stoffströme verhindert, daß sich im Aromastoff-Reservoir 33 Aerosolpartikel oder aus der Dampfphase gebildetes Kondensat, insbesondere Lösungsmittelkondensat absetzen können. Diese Abscheidungen könnten die Freisetzung des Aromastoffes empfindlich stören. Die beiden Stoffströme werden durch den vom Benutzer am Mundstück erzeugten Saugdruck induziert. Folglich sind die Strömungswiderstände des Aromastoff-Reservoirs 33 einerseits und des Kühlers 21 andererseits sowie gegebenenfalls weiterer durchströmter Komponenten so aufeinander abzustimmen, daß sich in Summe die gewünschte Widerstandscharakteristik, z.B. die einer Zigarette, und die gewünschten Durchflußraten einstellen.

Da dem Kühler 21 im konkreten Ausführungsbeispiel keine aromatisierende Wirkung abverlangt wird, liegt es nahe, diesen der Einfachheit halber einstufig auszuführen. Als Kühlermaterial kann wieder ein Synthetikfaservlies, wie bereits zuvor beschrieben, Verwendung finden. Das Aromastoff-Reservoir 33 kann beispielsweise aus einem durchströmbaren hochporösen Füllmaterial bestehen, wobei es besonders günstig ist, wenn das Füllmaterial Tabak enthält oder aus Tabak besteht. Als Tabak eignen sich insbesondere getrockneter fermentierter Tabak, rekonstituierter Tabak, expandierter Tabak oder Mischungen derselben. Besonders vorteilhaft ist es, wenn der Tabak als Schnitttabak, vorzugsweise Feinschnitttabak, oder als feines Granulat oder Tabakmehl vorliegt. Die große Oberfläche dieser Tabakformen unterstützt die Freisetzung der im Tabak enthaltenen Aromastoffe. Das Aromastoff-Reservoir 33 kann alternativ auch aus einem inerten Füllmaterial oder sonstigen offenporigen Trägermaterial bestehen, deren Oberfläche mit dem Aromastoff beschichtet ist. Die Beschichtung kann beispielsweise ein Tabakrauch-Kondensat oder ein Tabakextrakt oder eine flüchtige aromatische Fraktion der vorgenannten Stoffgemische oder ein Tabakmehl enthalten. Die Beschichtung kann alternativ oder ergänzend auch noch Menthol oder ein dieses beinhaltendes ätherisches Öl enthalten.

WO 2010/095659 (Takeuchi Manabu et al.) und WO 2010/095660 (Yamada Manabu et al.) beschreiben detaillierte Ausführungsbeispiele von luftdurchströmten Aromastoff-Reservoiren. Mit solchen Aromastoff-Reservoiren ausgestattete Inhalatoren sind mittlerweile auch bereits auf den Markt gekommen und werden von Japan Tobacco Inc., www.jt.com, unter dem Markennamen ZeroStyle® vertrieben.

Da sich die Riechstoff-Freisetzung im Ausführungsbeispiel nach Fig. 13 von jener nach Fig. 11 nicht unterscheidet, wurde auf eine nochmalige Darstellung dieser Zusammenhänge verzichtet.

Die Erfindung ist selbstverständlich nicht auf die anhand der Zeichnungen dargestellten Ausführungsbeispiele beschränkt und kann natürlich nach den jeweiligen Erfordernissen zweckmäßig weitergebildet werden. So wäre es zum Beispiel denkbar, außen am Gehäuse der Inhalatorkomponente 2 einen Schiebedeckel anzuordnen, mit dessen Hilfe die effektiv mit der Umgebung kommunizierende Fläche des Riechstoff-Reservoirs 6 variabel justiert werden kann, womit dem Benutzer die Möglichkeit geboten wäre, die Riechstoff-Freisetzung seinen persönlichen Bedürfnissen anzupassen. Der Schiebedeckel könnte auch dazu verwendet werden, das Riechstoff-Reservoir 6 komplett zu verschließen, beispielsweise während längerer Zeiten der Nichtbenutzung des Inhalators. Alternativ könnte auch eine über die Stirnfläche der Inhalatorkomponente 2 und über das Mundstück 4 übergestülpte und außen am Gehäuse 3 geführte Kappe 31 vorgesehen werden (siehe Fig. 1), welche nicht nur das Riechstoff-Reservoir 6, sondern gleichzeitig auch die Mundstücköffnung 5 verschließt. Um ein Entweichen des Riechstoffes 16 schon vor der eigentlichen Nutzung des Riechstoff-Reservoirs 6 auszuschließen, ist das Riechstoff-Reservoir 6 geeignet zu verpacken. Eine solche Verpackung kann auf einfache Weise durch eine selbstklebende impermeable Schutzfolie 32 bewirkt werden, welche das Riechstoff-Reservoir 6 flächig überdeckt (siehe Fig. 3; strichlierter Pfeil). Das auswechselbare Mundstück 4 als solches sollte in einer luftdichten Folienverpackung abgepackt in den Handel gelangen.

### Bezugszeichenliste

- 1: Inhalatorteil
- 2: Inhalatorkomponente
- 3: Gehäuse
- 3a: Gehäusekomponente
- 4: Mundstück
- 5: Mundstücköffnung
- 6: Riechstoff-Reservoir
- 7: Schnapphaken
- 8: Rastnase
- 9: Hohlzylinder
- 10: Öffnung
- 11: Mantelkomponente; erstes Mantelteil
- 12: Aussparung
- 13: Ausnehmung
- 14: Zylindermantel; zweites Mantelteil
- 15: Erhebung
- 16: Riechstoff
- 17: gasdurchlässige Membran
- 18: Riechstoff-Packung
- 19: impermeable Folie
- 20: Porenkörper
- 21: Kühler
- 22: Vorkühler
- 23: Tabakfüllung
- 24: Tabakstrang
- 25: Drahtgewebe
- 26: Mundstückkanal
- 27: Benutzer
- 28: Lippen
- 29: Nase
- 30: Nasenlöcher
- 31: Kappe
- 32: Schutzfolie
- 33: Aromastoff-Reservoir

## Patentansprüche

1. Inhalatorkomponente, aufweisend:
- ein Gehäuse (3) mit einem Gehäusemantel;
- ein Mundstück (4) mit einer Mundstücköffnung (5) für die Zufuhr eines inhalierbaren Mediums in die Mundhöhle eines Benutzers; wobei
a) das Gehäuse (3) eine Gehäusekomponente (3a) umfaßt;
b) das Mundstück (4) mit der Gehäusekomponente (3a) trennbar verbunden ist;
c) der Gehäusemantel ein erstes Mantelteil (11) und ein zweites Mantelteil (14) umfaßt;
d) die Gehäusekomponente (3a) das erste Mantelteil (11) ausbildet;
e) das Mundstück (4) das zweite Mantelteil (14) ausbildet,
f) und ein Riechstoff-Reservoir (6) mit dem Mundstück (4) baulich vereinigt ist, **dadurch gekennzeichnet, daß**
das Riechstoff-Reservoir (6) mit der Umgebung durch Diffusion kommunizierbar ist und einen Riechstoff (16) beinhaltet, zur Freisetzung des Riechstoffes (16) in die Umgebung und zur olfaktorischen Wahrnehmung desselben durch den Benutzer, und
das Riechstoff Reservoir (6) flach, platten- oder schalenförmig ausgebildet ist und außen flächig am zweiten Mantelteil (14) angeordnet ist oder selbst das zweite Mantelteil ausbildet.

2. Inhalatorkomponente **nach Anspruch 1, dadurch gekennzeichnet, daß** das Riechstoff-Reservoir (6) mit dem Mundstück (4) untrennbar verbunden ist.

3. Inhalatorkomponente **nach Anspruch 1 oder 2, dadurch gekennzeichnet, daß** das erste Mantelteil (11) und das zweite Mantelteil (14) sich im Bereich des Riechstoff-Reservoirs (6) oder/und in dessen Umfeld zumindest teilweise überlappen.

4. Inhalatorkomponente **nach einem der Ansprüche 1-3, dadurch gekennzeichnet, daß** das Riechstoff-Reservoir (6) oder/und der das Riechstoff-Reservoir aufnehmende Abschnitt des zweiten Mantelteils (14) zumindest teilweise in eine Ausparung (12) oder Einbuchtung des ersten Mantelteils (11) eingreift/eingreifen, oder das Riechstoff-Reservoir (6) über eine Ausparung (12) im ersten Mantelteil (11) mit der Umgebung kommuniziert.

5. Inhalatorkomponente **nach einem der Ansprüche 1-4** mit einer das Gehäuse (3) in Mantelrichtung etwa in zwei gleich große Teile spaltenden Gehäuse-Mittelebene m, **dadurch gekennzeichnet, daß** die Mundstücköffnung (5) und das Riechstoff-Reservoir (6) auf gegenüberliegenden Seiten der Gehäuse-Mittelebene m angeordnet sind.

6. Inhalatorkomponente **nach einem der Ansprüche 1-5, dadurch gekennzeichnet, daß** das Mundstück (4) außerdem einen von der Dampf- bzw. Partikelphase des inhalierbaren Mediums durchströmbaren Kühler (21) umfaßt.

7. Inhalatorkomponente **nach einem der Ansprüche 1-5, dadurch gekennzeichnet, daß** das Mundstück (4) außerdem ein das inhalierbare Medium mit Aromastoffen anreicherndes Aromastoff-Reservoir (33) umfaßt.

8. Inhalatorkomponente **nach Anspruch 7, dadurch gekennzeichnet, daß** das Aromastoff-Reservoir als ein von der Umgebungsluft durchströmbares, aber von der Dampf- bzw. Partikelphase des inhalierbaren Mediums unbeaufschlagtes Reservoir (33) ausgebildet ist.

9. Inhalatorkomponente **nach einem der Ansprüche 1-8, dadurch gekennzeichnet, daß** der Riechstoff (16) des Riechstoff-Reservoirs (6) Tabak enthält.

10. Inhalatorkomponente **nach Anspruch 9, dadurch gekennzeichnet, daß** der Tabak als Schnitttabak, vorzugsweise Feinschnitttabak, oder als feines Granulat oder Tabakmehl vorliegt.

11. Inhalatorkomponente **nach einem der Ansprüche 1-8, dadurch gekennzeichnet, daß** der Riechstoff (16) des Riechstoff-Reservoirs (6) ein Tabakrauch-Kondensat oder ein Tabakextrakt oder eine flüchtige aromatische Fraktion der vorgenannten Stoffgemische enthält; der Riechstoff (16) jedoch im Wesentlichen nikotinfrei ist.

12. Inhalatorkomponente **nach Anspruch 11, dadurch gekennzeichnet, daß** der Riechstoff (16) des Riechstoff-Reservoirs (6) wenigstens eine flüchtige Säure enthält und der Anteil aller flüchtigen Säuren im Riechstoff (16) in Summe größer als 5 Massen-% ist.

13. Inhalatorkomponente **nach einem der Ansprüche 9-12, dadurch gekennzeichnet, daß** der Riechstoff (16) des Riechstoff-Reservoirs (6) Menthol enthält.

14. Auswechselbares Mundstück (4) für die Verwendung mit einer Inhalator-Komponente nach einem der Ansprüche 1 bis 13, wobei
- ein Riechstoff-Reservoir (6) mit dem Mundstück (4) baulich vereinigt ist,
- das Mundstück eine Mundstücköffnung (5) für die Zufuhr eines inhalierbaren Mediums in die Mundhöhle eines Benutzers aufweist,
- das Mundstück als ein zweites Mantelteil (14) zur Anordnung am ersten zur Inhalator-Komponente gehörenden Mantelteil (11) ausgebildet ist,
**dadurch gekennzeichnet, daß**
- das Riechstoff-Reservoir (6) mit der Umgebung durch Diffusion kommunizierbar ist und einen Riechstoff (16) beinhaltet, zur Freisetzung des Riechstoffes (16) in die Umgebung und zur olfaktorischen Wahrnehmung desselben durch den Benutzer, und
- das Riechstoff Reservoir (6) flach, platten- oder schalenförmig ausgebildet ist und außen flächig am zweiten Mantelteil angeordnet ist oder selbst das zweite Mantelteil ausbildet.

15. Inhalator, umfassend eine Inhalatorkomponente (2) **nach einem der Ansprüche 1-13**.

## Claims

1. Inhaler component, comprising:
- a housing (3) with a housing jacket;
- a mouthpiece (4) with a mouthpiece opening (5) for supplying an inhalable medium into the oral cavity of a user; whereby
a) the housing (3) comprises a housing component (3a);
b) the mouthpiece (4) is connected to the housing component (3a) such that they can be separated;
c) the housing jacket comprises a first jacket part (11) and a second jacket part (14);
d) the housing component (3a) forms the first jacket part (11) ;
e) the mouthpiece (4) forms the second jacket part (14);
f) and an odorant reservoir (6) and the mouthpiece (4) are joined by constructive means;
**characterised in that**
the odorant reservoir (6) can communicate with the surroundings by means of diffusion and contains an odorant (16) for release of the odorant (16) into the surroundings and for olfactory perception thereof by the user
and
the odorant reservoir (6) is formed to be flat, plate- or cup-shaped and is arranged on the outer surface of the second jacket part (14) or itself forms the second jacket part.

2. Inhaler component **according to claim 1, characterised in that** the odorant reservoir (6) is connected inseparably to the mouthpiece (4).

3. Inhaler component **according to claim 1 or 2, characterised in that** the first jacket part (11) and the second jacket part (14) overlap, at least in part, in the region of the odorant reservoir (6) or/and in its surroundings.

4. Inhaler component **according to any one of the claims 1-3, characterised in that** the odorant reservoir (6) or/and the section of the second jacket part (14) accommodating the odorant reservoir engage(s), at least in part, a recess (12) or indentation of the first jacket part (11), or the odorant reservoir (6) communicates with the surroundings by means of a recess (12) in the first jacket part (11).

5. Inhaler component **according to any one of the claims 1-4** having a central plane m of the housing that cleaves the housing (3) in the direction of the jacket into two approximately equal-sized parts, **characterised in that** the mouthpiece opening (5) and the odorant reservoir (6) are arranged on opposite sides of the central plane m of the housing.

6. Inhaler component **according to any one of the claims 1-5, characterised in that** the mouthpiece (4) further comprises a cooler (21) through which the vapour and/or particle phase of the inhalable medium can flow.

7. Inhaler component **according to any one of the claims 1-5, characterised in that** the mouthpiece (4) further comprises an aromatic substance reservoir (33) that enriches the inhalable medium in aromatic substances.

8. Inhaler component **according to claim 7, characterised in that** the aromatic substance reservoir is formed as a reservoir (33) through which the ambient air can flow, but to which the vapour and/or particle phase of the inhalable medium is not applied.

9. Inhaler component **according to any one of the claims 1-8, characterised in that** the odorant (16) of the odorant reservoir (6) contains tobacco.

10. Inhaler component **according to claim 9, characterised in that** the tobacco is present as cut tobacco, preferably as fine-cut tobacco, or as a fine granulate or tobacco powder.

11. Inhaler component **according to any one of the claims 1-8, characterised in that** the odorant (16) of the odorant reservoir (6) contains a tobacco smoke condensate or a tobacco extract or a volatile aromatic fraction of the afore-mentioned mixtures of substances, while the odorant (16) is essentially free of nicotine though.

12. Inhaler component **according to claim 11, characterised in that** the odorant (16) of the odorant reservoir (6) contains at least one volatile acid and **in that** the fractions of all volatile acids in the odorant (16) add up to more than 5 % by mass.

13. Inhaler component **according to any one of the claims 9-12, characterised in that** the odorant (16) of the odorant reservoir (6) contains menthol.

14. Exchangeable mouthpiece (4) for use with an inhaler component according to any one of the claims 1 to 13, whereby
- an odorant reservoir (6) and the mouthpiece (4) are joined by constructive means;
- the mouthpiece comprises a mouthpiece opening (5) for supplying an inhalable medium into the oral cavity of a user;
- the mouthpiece is formed as a second jacket part (14) to be arranged on the first jacket part (11) that belongs to the inhaler component;
**characterised in that**
- the odorant reservoir (6) can communicate with the surroundings by means of diffusion and contains an odorant (16) for release of the odorant (16) into the surroundings and for olfactory perception thereof by the user
and
- the odorant reservoir (6) is formed to be flat, plate- or cup-shaped and is arranged on the outer surface of the second jacket part or itself forms the second jacket part.

15. Inhaler, comprising an inhaler component (2) **according to any one of the claims 1-13**.

## Revendications

1. Composant d'inhalateur présentant :
- un logement (3) avec une enveloppe de logement ;
- un embout (4) avec une ouverture d'embout (5) pour l'amenée d'un milieu inhalable dans la cavité buccale d'un utilisateur ; dans lequel
a) le logement (3) comprend un composant de logement (3a) ;
b) l'embout (4) est relié de manière à pouvoir être séparé au composant de logement (3a) ;
c) l'enveloppe de logement comprend une première partie d'enveloppe (11) et une seconde partie d'enveloppe (14) ;
d) le composant de logement (3a) constitue la première partie d'enveloppe (11) ;
e) l'embout (4) constitue la seconde partie d'enveloppe (14),
f) et un réservoir de substance odorante (6) est uni à l'embout (4) au niveau de la construction,
**caractérisé en ce que**
le réservoir de substance odorante (6) peut communiquer avec l'environnement par diffusion et contient une substance odorante (16), pour la libération de la substance odorante (16) dans l'environnement et pour la perception olfactive de celle-ci par l'utilisateur, et
le réservoir de substance odorante (6) est réalisé de manière plate, en forme de plaque ou de coque et est disposé à l'extérieur de manière plane sur la seconde partie d'enveloppe (14) ou constitue lui-même la seconde partie d'enveloppe.

2. Composant d'inhalateur **selon la revendication 1, caractérisé en ce que** le réservoir de substance odorante (6) est relié de manière à ne pas pouvoir être séparé à l'embout (4).

3. Composant d'inhalateur **selon la revendication 1 ou 2, caractérisé en ce que** la première partie d'enveloppe (11) et la seconde partie d'enveloppe (14) se chevauchent au moins partiellement dans la région du réservoir de substance odorante (6) ou/et dans son environnement.

4. Composant d'inhalateur **selon une des revendications 1 à 3, caractérisé en ce que** le réservoir de substance odorante (6) ou/et la section de la seconde partie d'enveloppe (14) recevant le réservoir de substance odorante s'engage(nt) au moins partiellement dans un évidement (12) ou une échancrure de la première partie d'enveloppe (11), ou le réservoir de substance odorante (6) communique avec l'environnement par le biais d'un évidement (12) dans la première partie d'enveloppe (11).

5. Composant d'inhalateur **selon une des revendications 1 à 4** avec un plan médian de logement m divisant le logement (3) dans le sens de l'enveloppe approximativement en deux parties de même taille, **caractérisé en ce que** l'ouverture d'embout (5) et le réservoir de substance odorante (6) sont disposés sur des côtés opposés du plan médian de logement m.

6. Composant d'inhalateur **selon une des revendications 1 à 5, caractérisé en ce que** l'embout (4) comprend en outre un réfrigérant (21) pouvant être parcouru par la phase de vapeur ou particulaire du milieu inhalable.

7. Composant d'inhalateur **selon une des revendications 1 à 5, caractérisé en ce que** l'embout (4) comprend en outre un réservoir de substance aromatisante (33) enrichissant en substances aromatisantes le milieu inhalable.

8. Composant d'inhalateur **selon la revendication 7, caractérisé en ce que** le réservoir de substance aromatisante est réalisé en tant que réservoir (33) pouvant être parcouru par l'air ambiant, mais non sollicité par la phase de vapeur ou particulaire du milieu inhalable.

9. Composant d'inhalateur **selon une des revendications 1 à 8, caractérisé en ce que** la substance odorante (16) du réservoir de substance odorante (6) contient du tabac.

10. Composant d'inhalateur **selon la revendication 9, caractérisé en ce que** le tabac est présent en tant que tabac haché, de préférence tabac haché finement, ou en tant que granulat fin ou farine de tabac.

11. Composant d'inhalateur **selon une des revendications 1 à 8, caractérisé en ce que** la substance odorante (16) du réservoir de substance odorante (6) contient un condensé de fumée de tabac ou un extrait de tabac ou une fraction aromatique volatile des mélanges de substances susmentionnés ; la substance odorante (16) est toutefois essentiellement exempte de nicotine.

12. Composant d'inhalateur **selon la revendication 11, caractérisé en ce que** la substance odorante (16) du réservoir de substance odorante (6) contient au moins un acide volatile et la proportion de tous les acides volatiles dans la substance odorante (16) est au total supérieure à 5 % en masse.

13. Composant d'inhalateur **selon une des revendications 9 à 12, caractérisé en ce que** la substance odorante (16) du réservoir de substance odorante (6) contient du menthol.

14. Embout échangeable (4) pour l'utilisation avec un composant d'inhalateur selon une des revendications 1 à 13, dans lequel
- un réservoir de substance odorante (6) est uni à l'embout (4) au niveau de la construction,
- l'embout présente une ouverture d'embout (5) pour l'amenée d'un milieu inhalable dans la cavité buccale d'un utilisateur,
- l'embout est réalisé en tant qu'une seconde partie d'enveloppe (14) pour la disposition sur la première partie d'enveloppe (11) appartenant au composant d'inhalateur,
**caractérisé en ce que**
- le réservoir de substance odorante (6) peut communiquer avec l'environnement par diffusion et contient une substance odorante (16), pour la libération de la substance odorante (16) dans l'environnement et pour la perception olfactive de celle-ci par l'utilisateur, et
- le réservoir de substance odorante (6) est réalisé de manière plate, en forme de plaque ou de coque et est disposé à l'extérieur de manière plane sur la seconde partie d'enveloppe ou constitue lui-même la seconde partie d'enveloppe.

15. Inhalateur comprenant un composant d'inhalateur (2) **selon une des revendications 1 à 13.**
